# EUROPEAN PATENT SPECIFICATION

(11) **EP 4 623 296 B1**
(45) Date of publication and mention of the grant of the patent: **14.10.2026**
(21) Application number: 24837352.4
(22) Date of filing: 19.12.2024
(51) Int. Cl.: G01N 33/24, G01N 27/22, H02J 50/20, H02J 50/80, H04Q 9/00

(54) **MOISTURE SENSOR, MEASURING METHODS AND USES**
FEUCHTIGKEITSSENSOR, MESSVERFAHREN UND VERWENDUNGEN
CAPTEUR D'HUMIDITÉ, PROCÉDÉS DE MESURE ET UTILISATIONS

(30) Priority: 20.12.2023 DE 102023136045; 20.12.2023 EP 23218420
(43) Date of publication of application: 01.10.2025
(73) Proprietor: BASF Agricultural Solutions US LLC, Research Triangle Park, NC 27713 (US)
(72) Inventor: BLASZKIEWICZ, Kamil, 9052 Gent (BE)
(74) Representative: AP-IP
(86) International application number: PCT/EP2024/087568
(87) International publication number: WO 2025/132899

(56) References cited:
- WO-A1-2022/160069
- KR-A- 20170 115 267
- US-A1- 2004 078 662
- US-A1- 2011 018 686
- US-A1- 2011 287 713
- US-A1- 2018 084 314
- US-B1- 10 499 573
- LE MINH THUY ET AL: "Wireless Powered Moisture Sensors for Smart Agriculture and Pollution Prevention: Opportunities, Challenges, and Future Outlook", vol. 9, no. 4, 1 December 2023 (2023-12-01), pages 646 - 659, XP009554025, ISSN: 2198-6592, Retrieved from the Internet <URL:https://link.springer.com/article/10.1007/s40726-023-00286-3> DOI: 10.1007/S40726-023-00286-3
- SHENG DING: "Wireless Power Transfer Through Soil for Energizing an Underground Soil Moisture Sensor", 2022 IEEE SENSORS, 30 October 2022 (2022-10-30), pages 1 - 4, XP093161334, ISBN: 978-1-6654-8464-0, DOI: 10.1109/SENSORS52175.2022.9967363
- CHARLES A. ROBINSON: "Development of A Wireless Power Transmission System for Agriculture Sensor Devices", 2020 IEEE INTERNATIONAL CONFERENCE ON BIG DATA (BIG DATA), 10 December 2020 (2020-12-10), pages 2360 - 2364, XP093161341, ISBN: 978-1-7281-6251-5, DOI: 10.1109/BigData50022.2020.9377855
- XIAOLIN FANG: "Energy Efficient Data Transmission for Sensors with Wireless Charging", SENSORS, vol. 18, no. 2, 8 February 2018 (2018-02-08), CH, pages 511, XP093161637, ISSN: 1424-8220, DOI: 10.3390/s18020511

## Description

### TECHNICAL FIELD

The present invention relates generally to moisture sensors, in particular to moisture sensors using time domain reflectometry (TDR). In particular, the invention concerns a sensor device which is charged using wireless power transfer and subsequently performs a moisture measurement using time domain reflectometry. The invention also provides measuring methods using such sensor device and uses or application methods which employ such sensor device.

### BACKGROUND

Measuring moisture of a sample, preferably a solid sample, more preferably soil, is an important aspect in numerous fields, such as geology, engineering, construction, biology, and agriculture. For instance, in agriculture, managing plant growth while being confronted with increasingly limited water resources due to climate change is an important topic. This requires a continuous monitoring of the soil moisture. The term "soil" refers to the layer(s) of generally loose mineral and/or organic material that are affected by physical, chemical, and/or biological processes at or near the planetary surface and usually hold liquids, gases, and biota and support plants. It serves as a medium for plant growth, providing to plant roots nutrients, water and anchorage.

The methods to monitor moisture can be categorized in direct and indirect methods. Direct methods measure the water content of a sample by removing the water through evaporation or chemical reaction. Examples of direct methods are the gravimetric method and the calcium carbide method. Indirect methods measure a property of the sample that fluctuates with water content, such as dielectric constant, electrical conductivity or optical reflectance. Examples of indirect methods are time domain reflectometry (TDR), time domain transmissometry (TDT), electrical conductivity, and near-infrared optical methods.

The principle of Time Domain Reflectometry (TDR) is founded on the relationship between the dielectric constant of a sample and its water content. A TDR sensor transmits one or more highfrequency electromagnetic pulses through waveguides and measures the time delay between the incident and reflected electromagnetic pulses. These waveguides typically consist of a pair of stainless-steel rods inserted into the sample. The significant difference between the dielectric constant of water, which is approximately 80, and that of other materials, which ranges from 2 to 7, results in pulse travel time that is dependent on the volumetric moisture content.

US20210051867 describes the measurement of volumetric water content using TDR but does not address the power supply requirements for the TDR sensor. There are several methods for powering a TDR sensor: wired connections (as illustrated in WO2004074781 figures 1 and 2, and US9,696,292 figures 1-3A) can be impractical in agricultural settings, as they may obstruct the movement of plant pots in greenhouses, particularly when conveyor belts are employed. Similarly, TDR sensors powered by bulky batteries pose stability issues, leading to the potential toppling of moving plant pots and limiting the ability to capture images of the plants due to the battery size. Additionally, the use of batteries incurs high maintenance costs associated with regular replacements.

Alternatively, TDR sensors may potentially be powered wirelessly. Fonsêca et al. developed a passive RFID soil moisture sensor that employs Time Domain Transmissometry (TDT) (N. Fonseca, R. Freire, G. Fontgalland, B. Arruda, and S. Tedjini, "A Fully Passive UHF RFID Soil Moisture Time-Domain Transmissometry Based Sensor," 2018 3rd International Symposium on Instrumentation Systems, Circuits and Transducers (INSCIT), Bento Gongalves, Brazil, 2018, pp. 1-6). This paper indicates that measurement is achieved by simultaneously sampling the injected and propagated signals to determine the propagation delay time. However, this method is not suitable for TDR measurements because electromagnetic radiation can introduce unwanted noise and interference into the TDR system, potentially compromising measurement accuracy and reliability by distorting reflected signals and complicating the differentiation between desired reflections and extraneous noise. Furthermore, as noted in the study by Sun et al. (Sun and G. D. Young, "A Cost Effective Soil Moisture Instrument Based on Time-Domain Transmission Measurement," 2001), TDT does not perform well under saline clay soil conditions, for example.

LE MINH THUY ET AL entitled: "Wireless Powered Moisture Sensors for Smart Agriculture and Pollution Prevention: Opportunities, Challenges, and Future Outlook", published in CURRENT POLLUTION REPORTS, SPRINGER, vol. 9, no. 4 1 December 2023 (2023-12-01), pages 646-659, ISSN: 2198-6592, DOI: 10.1007/S40726-023-00286-3 provides a review which evaluates available soil moisture sensing and wireless power transfer techniques for wireless under-ground moisture sensing.

Therefore, the present invention aims to provide a sensor that mitigates or eliminates one or more of the aforementioned disadvantages of the prior art.

### SUMMARY OF THE INVENTION

The invention provides a sensor device, the sensor device comprising:
an antenna for receiving electromagnetic radiation;
a capacitor configured to store power received from the antenna;
a threshold activation element electrically connected to the capacitor to compare the stored power to a predefined threshold, wherein the predefined threshold is sufficient to enable the generation of time domain reflectometry (TDR) sensor data;
a detection element for detecting the interruption of power supply, and
a TDR sensor to generate TDR sensor data when both the threshold activation element signaled that the stored power in the capacitor exceeds the predefined threshold, and the detection element signaled that the antenna has ceased receiving electromagnetic radiation.

The invention provides a sensor device operation method, comprising the steps of:
i) providing a sensor device,
ii) powering the sensor device by electromagnetic radiation from an emitter,
iii) determining, in the sensor device, if the sensor device is sufficiently powered to conduct a TDR measurement and if transmission of electromagnetic radiation to the device has been interrupted, and
iv) optionally, when it is determined in step iii) that the sensor device is sufficiently powered to conduct the TDR measurement, preventing further powering of the sensor device by electromagnetic radiation from the emitter, and
(v) performing a TDR measurement generating TDR sensor data if both conditions of step (iii) are fulfilled or alternatively if the condition of step (iv) is fulfilled.

The invention provides an automated greenhouse, comprising
- a container, preferably a container for containing one or more plants, comprising solid material, and a sensor device for determining the moisture of the solid material,
- an emitter and
- a recorder,
further comprising conveyor means to bring the antenna of the sensor device in the container into a power transmission distance to the emitter and signal exchange distance to the recorder.

### BRIEF DESCRIPTION OF THE FIGURES

Figure 1 illustrates an example of a sensor device (100) for performing time domain reflectometry (TDR) measurements of a solid material.
Figure 2 illustrates an example of a sensor device (200) for performing time domain reflectometry (TDR) measurements of a solid material comprising an integrated circuit (210).
Figure 3 illustrates an example of a sensor device (300) for performing time domain reflectometry (TDR) measurements of a solid material comprising a transmitter (310).
Figure 4 illustrates an example of a sensor device (400) for performing time domain reflectometry (TDR) measurements of a solid material comprising an integrated circuit (210) and a transmitter (310).
Figure 5 illustrates an example of a sensor device operation method.
Figure 6 illustrates an example of a sensor device operation method wherein further powering of the sensor device by electromagnetic radiation is prevented.
Figure 7 illustrates an example of a sensor device operation method wherein the measurement data is recorded.

### DETAILED DESCRIPTION

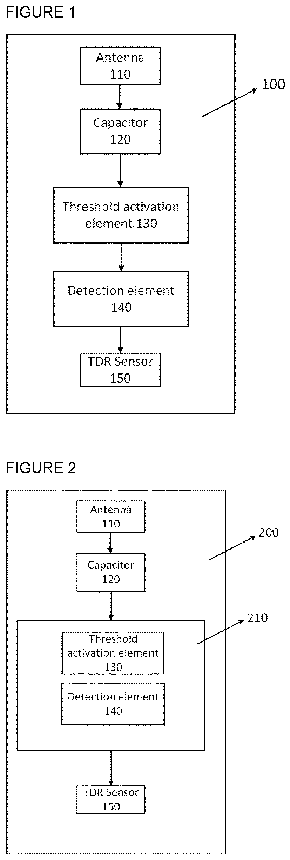

As illustrated in Figure 1, the invention concerns a sensor device (100) in accordance with independent claim 1 comprising the following elements: an antenna (110), a capacitor (120), a threshold activation element (130), a detection element (140), and a TDR sensor (150).

The antenna (110) is a transducer that converts electric current into electromagnetic waves and vice versa. Antennas can transmit and receive electromagnetic fields, preferably radio waves and/or microwaves.

The capacitor (120) is electrically connected to the antenna (110) and configured to store power received from the antenna (110). The capacitor (120) is thus charged using power received via the antenna (110).

The threshold activation element (130) is electrically connected to the capacitor (120) which is configured to compare the stored power to a predefined threshold. The threshold activation element (130) is configured such that the predefined threshold is sufficient to enable the generation of time domain reflectometry (TDR) sensor data. The threshold activation element (130) may be a comparator. The threshold activation element (130) may be a digital comparator, analog comparator, transistor switch or a microcontroller with analog-to-digital (ADCs) converters.

The detection element (140) is configured for identifying the cessation of receiving electromagnetic radiation. The detection element (140) may be a comparator, wherein the comparator activates the TDR sensor (150) after stored power in the capacitor (120) falls below the predefined threshold of the comparator due to the termination of receiving electromagnetic radiation. The detection element (140) may be an analog comparator or a digital comparator. The detection element (140) may be a transistor switch. The detection element (140) may be a relay. The detection element (140) may be a voltmeter integrated with a comparator, providing feedback on voltage levels. The detection element (140) may be an ammeter integrated with a comparator, providing feedback on the current. The detection element (140) may be a microcontroller with analog-to-digital (ADCs) converters.

The TDR sensor (150) is configured to generate TDR sensor data. The TDR sensor (150) generates TDR sensor data when both the threshold activation element (130) signals that the stored power in the capacitor (120) exceeds the predefined threshold, and the detection element (140) signals that the antenna (110) has ceased receiving electromagnetic radiation. In other words, the sensor device (100) starts a measurement after the capacitor (120) is sufficiently charged and the antenna (110) does not receive any electromagnetic radiation to charge the capacitor (120). The TDR sensor (150) is thus capable of sensing the moisture of a solid sample, preferably a soil sample, when the TDR sensor (150) is inserted therein. The TDR sensor (150) of the sensor device (100) preferably comprises at least two rods: a reference rod and at least a measuring rod. Additionally, it is preferable for the TDR sensor (150) to contain only one reference rod but multiple measuring rods. Preferably, all measuring rods are coated with electrically insulating material and the reference rod is not. Preferably, multiple measuring rods have different length which allows to measure moisture in function of the depth when the rods are inserted in a solid material, e.g. soil.

The sensor device (100) of the present invention offers a number of advantages over prior art: The sensor device (100) allows to generate TDR sensor data without the need of wires. In particular, the sensor device (100) of the present invention can be operated without power cables and batteries. Therefore, the sensor device (100) can monitor soil moisture in an agricultural field without the need of power cables. In a greenhouse, there is no need of power cables or batteries which obstruct the view of a plant growing in a plant pot comprising the sensor device (100) of the present invention. This design allows for continuous monitoring and imaging of the plants without the need to remove the sensor device (100). Additionally, the sensor device (100) minimizes disturbances to the TDR sensor (150) from electromagnetic radiation emitted by external sources such as an emitter (500), which can introduce unwanted noise and interference into the TDR system. Such interference could compromise measurement accuracy and reliability by distorting reflected signals and making it difficult to distinguish between desired reflections and extraneous noise.

The sensor device (100) preferably comprises a temperature sensor to measure the temperature of the solid material. The temperature sensor is electrically connected to the threshold activation element (130) such as to generate temperature sensor data only when the stored power in the capacitor (120) exceeds the predetermined threshold. Preferably, the temperature of the solid material is used to calibrate the TDR measurements. The sensor device (100) of the present invention can perform such TDR sensor data correction internally, based on a stored mathematical function or by interpolation using a lookup table.

In one embodiment, the sensor device (200) includes an integrated circuit (210), as illustrated in Figure 2. The term "integrated circuit" refers to a compact electronic device that contains multiple interconnected electronic components, such as transistors, capacitors, resistors, and diodes, all on a single chip or substrate. These components are arranged and interconnected in a way that allows the integrated circuit to perform specific electronic functions, such as processing, storage, and communication. The term "integrated circuit" also includes a microcontroller, which is a specialized integrated circuit that combines a central processing unit (CPU), memory, and input/output (I/O) peripherals on a single chip. The integrated circuit (210) is electrically connected to the threshold activation element (130) such that the integrated circuit (210) or microcontroller is powered only when sufficient power is stored in the capacitor (120). The integrated circuit (210) or microcontroller is also connected to the detection element (140) and the TDR sensor (150). Preferably, the integrated circuit (210) stores the TDR sensor data of the TDR sensor (150) and/or temperature data of the temperature sensor. Preferably, if the sensor device (200) contains multiple measuring rods, the integrated circuit (210) stores in an internal memory, after each TDR measurement, an indicator for identifying the rod to be used by the TDR sensor (150) for the next TDR measurement.

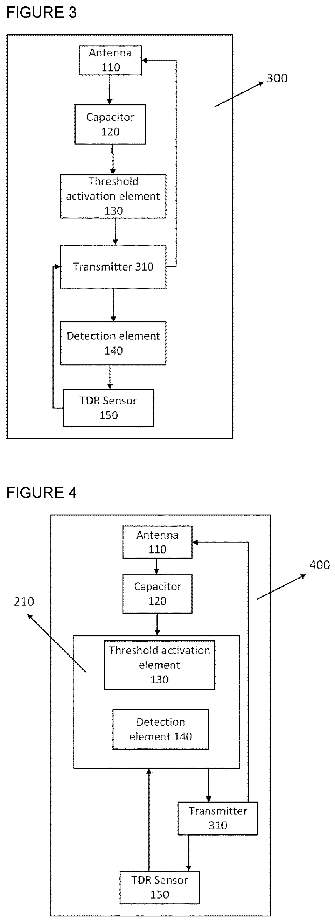

In another embodiment, the sensor device (300) comprises a transmitter (310), as illustrated in Figure 3. The transmitter (310) is configured to generate a digital data signal and wherein the antenna (110) is electrically connected to the transmitter (310) to emit the digital data signal. In one embodiment, the transmitter (310) converts the signals produced by the TDR sensor (150) and/or temperature sensor into a digital data signal and the antenna (110) is configured to emit the digital data signal (410). The digital data signal may include TDR sensor data that has been corrected using the temperature data. Preferably, the transmitter (310) is electrically connected to the temperature sensor. The sensor device (300) of the present invention can transmit the digital data signal (410) based on uncorrected TDR sensor data and temperature sensor data. The TDR sensor data can be corrected on a recorder side having received the digital data signal (410) comprising both the uncorrected TDR sensor data and the temperature sensor data; such correction can preferably be performed by using a mathematical function fitted to pre-recorded temperature calibration data, or it can be performed by interpolation in a lookup table of pre-recorded temperature calibration data. Preferably, the sensor device (300) of the present invention emits both the uncorrected TDR sensor data and the temperature sensor data without performing a temperature correction. This way, the amount of energy consumed by the sensor device (300) to perform a moisture content measurement and transmit the measurement data is minimized.

In another embodiment, the transmitter (310) is configured to generate a blocking signal (620) which is emitted by the antenna (110) once the predefined threshold of the threshold activation element (130) is reached. The purpose of this blocking signal (620) is to cease the emitter's (500) transmission of electromagnetic radiation, thereby preventing the antenna (110) from receiving any additional electromagnetic radiation from the emitter (500). Preferably, the blocking signal may serve as a feedback mechanism for the emitter when, upon receiving this signal, the emitter's internal systems can interpret it as a command to halt transmission.

In another embodiment, the sensor device (400) comprises both an integrated circuit (210) and a transmitter (310). The integrated circuit (210) processes the data collected from the TDR sensor (150) and/or the temperature sensor. The transmitter (310) generates a digital data signal for transmission. This configuration allows the device to efficiently emit both TDR sensor data and/or temperature data to external systems, such as a recorder (700), for analysis.

Preferably, the TDR sensor (150) comprises a time-to-digital converter (TDC). A TDC is used to accurately measure the time delay between start and stop signals and convert it into a digital output. A TDC can measure the time delay between the start and stop signals of the reflected pulse of the TDR sensor (150) with high resolution, providing accurate measurements. Using a TDC offers the advantage of easy integration within the sensor device (100), which in turn, contributes to the miniaturization of the TDR sensor (150). Additionally, miniaturization of the TDR sensor (150) enables its integration into plant pots within a greenhouse. This integration allows for imaging of plants in the pots without any obstruction from the sensor device (100).

Preferably, the transmitter (310) of the sensor device (300) comprises an RFID chip or NFC chip. The sensor device (300) with RFID chip or NFC chip functions as a tag or label to identify the measured solid material.

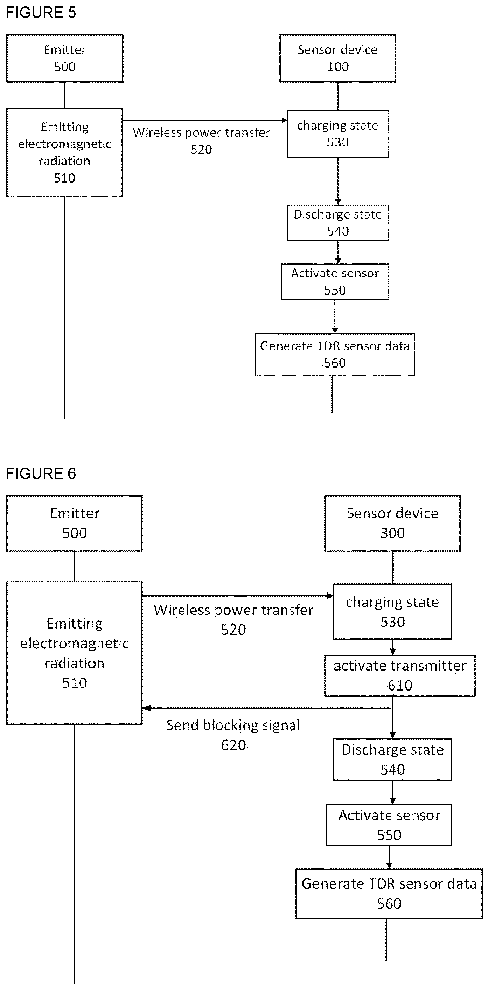

An example of a sensor device operation method to measure a sensor signal is illustrated in Figure 5. The emitter (500) emits electromagnetic radiation (510) to the sensor device (100) and the antenna (110) of the sensor device (100) converts the electromagnetic radiation into electric current which charges the capacitor (120) if the emitter (500) is in a power transmittable range of the antenna (110). This process is known as wireless power transfer (520). Wireless power transfer (520) is the process of transmitting energy by electromagnetic radiation between an emitter (500) and one or more receivers, such as the sensor device (100). During a charging state (530), the capacitor (120) accumulates electrical energy until the predefined threshold of the threshold activation element (130) is reached. Once the predefined threshold is reached, the threshold activation element (130) activates the detection element (140). When the capacitor (120) is no longer charged, it indicates that the emitter (500) has ceased emitting electromagnetic radiation, placing the sensor device (100) in a discharge state (540). If the detection element (140) identifies the interruption of power supply-indicated by the capacitor (120) no longer being charged due to the emitter (500) stopping its transmission of electromagnetic radiation-it activates (550) the TDR sensor (150) to generate TDR sensor data (560).

An example of a sensor device operation method to measure a sensor signal wherein further powering of the sensor device (300) by electromagnetic radiation is prevented is illustrated in Figure 6. The emitter (500) emits electromagnetic radiation (510) to the sensor device (300) and the antenna (110) of the sensor device (300) converts the electromagnetic radiation into electric current which charges the capacitor (120) if the emitter (500) is in a power transmittable range of the antenna (110). This process is known as wireless power transfer (520). During a charging state (530), the capacitor (120) accumulates electrical energy until the predefined threshold of the threshold activation element (130) is reached. Once the predefined threshold is reached, the threshold activation element (130) activates the transmitter (310) and the detection element (140). The transmitter (310) generates a blocking signal (620) which is emitted by the antenna (110). The purpose of this blocking signal (620) is to cease the emitter's (500) transmission of electromagnetic radiation, thereby preventing the antenna (110) from receiving any additional electromagnetic radiation from the emitter (500). When the capacitor (120) is no longer charged, it indicates that the emitter (500) has ceased emitting electromagnetic radiation, placing the sensor device (300) in a discharge state (540). If the detection element (140) identifies the interruption of power supply-indicated by the capacitor (120) no longer being charged due to the emitter (500) stopping its transmission of electromagnetic radiation-it activates (550) the TDR sensor (150) to generate TDR sensor data (560). Preferably, the transmitter (310) converts the signals produced by the TDR sensor (150) and/or temperature sensor into a digital data signal and the antenna (110) is configured to emit the digital data signal (410).

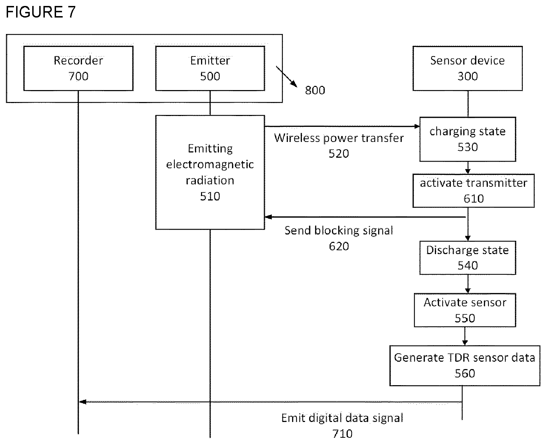

An example of a sensor device operation method wherein the measurement data is recorded is illustrated in Figure 7. The emitter (500) emits electromagnetic radiation (510) to the sensor device (300) and the antenna (110) of the sensor device (300) converts the electromagnetic radiation into electric current which charges the capacitor (120) if the emitter (500) is in a power transmittable range of the antenna (110). This process is known as wireless power transfer (520). During a charging state (530), the capacitor (120) accumulates electrical energy until the predefined threshold of the threshold activation element (130) is reached. Once the predefined threshold is reached, the threshold activation element (130) activates the transmitter (310) and the detection element (140). The transmitter (310) generates a blocking signal (620) which is emitted by the antenna (110). The purpose of this blocking signal (620) is to cease the emitter's (500) transmission of electromagnetic radiation, thereby preventing the antenna (110) from receiving any additional electromagnetic radiation from the emitter (500). When the capacitor (120) is no longer charged, it indicates that the emitter (500) has ceased emitting electromagnetic radiation, placing the sensor device (300) in a discharge state (540). If the detection element (140) identifies the interruption of power supply-indicated by the capacitor (120) no longer being charged due to the emitter (500) stopping its transmission of electromagnetic radiation-it activates (550) the TDR sensor (150) to generate TDR sensor data (560). The transmitter (310) converts the signals produced by the TDR sensor (150) and/or temperature sensor into a digital data signal and the antenna (110) is configured to emit the digital data signal (710). The antenna (110) of the sensor device (300) emits the digital data signal (710), which can be received by a recorder (700). Preferably, a reader (800) is used to both perform wireless power transfer (520) to charge the sensor device (300) and receive the emitted digital data signal of the sensor device (300). An example of a reader is an RFID reader or an NFC reader.

In another embodiment, the sensor device is equipped with a timer, which is programmed such that the sensor device enters a low-power state once the sensor device is fully charged, and only starts a TDR measurement after a set delay. This configuration enables the emitter and the device to be positioned in close proximity for wireless power transfer during a predetermined period, the predetermined period being a period to charge the device in order to be able to perform a TDR measurement. Once the emitter (500) is no longer in range, the sensor device will conduct the TDR measurement, after the specified delay, thereby preventing the antenna (110) from receiving any additional electromagnetic radiation from the emitter (500) during the TDR measurement. Preferably, a conveyor belt conveys the emitter and/or device from one location to another. The conveyor may include various components such as belts, rollers, chains, or other elements that facilitate the movement of the transported items.

Additionally, the invention provides a method in accordance with independent claim 7 which may be adapted to monitor moisture of a solid material in a container, preferably a container for containing one or more plants. The term container is meant herein to designate a receptacle capable of holding, storing, or transporting solid material. The container may have various shapes, sizes, and configurations and may be constructed from a variety of materials. The container thus comprises the solid material and a sensor device (300) of the present invention, wherein the rods of the sensor device (300) are inserted in the solid material. Preferably, the container comprising the solid material and the sensor device (300) is conveyed in a power transmittable range of an emitter (500) or vice versa. Preferably, a conveyor belt conveys the container or emitter (500) from one location to another. The conveyor may include various components such as belts, rollers, chains, or other elements that facilitate the movement of the transported items. The conveyor may be powered by motors or other means and may include sensors or control systems to regulate its operation. Subsequently, once the emitter (500) and sensor device (300) are in power transmittable range, the sensor device (300) is charged due to the emittance of electromagnetic radiation of the emitter (500) and a TDR measurement is performed as described earlier. A recorder (700) records a digital data signal (710) emitted by the sensor device (300) and a computing node compares the data with previously recorded data. Preferably, a reader (800), such as an RFID reader or NFC reader, is used which acts both as emitter (500) and recorder (700). The recorder (700) is connected to a computing node, which is configured to compare the TDR sensor data obtained by the sensor signal with TDR sensor data obtained with a previously recorded signal. Preferably, the sensor device (300) comprises an RFID chip or NFC chip to identify the solid material in the container.

The method provides an automated solution to monitor the moisture content of a solid material in a container, specifically in containers for holding plants within an automated greenhouse. By using the sensor device (300) integrated with a reader (800), such as an RFID reader or NFC reader, the system can accurately and efficiently monitor the moisture content of a solid material, providing valuable data for optimal plant growth and yield. It is particularly preferred that the automated greenhouse comprises a watering station which is configured with a computing node. The computing node monitors the moisture of the solid material in the plant pots and decides, based on the TDR sensor data, that the plant pot needs watering.

The invention also provides an automated greenhouse in accordance with independent claim 10. An automated greenhouse according to the present invention is a greenhouse fitted with means for automatically monitoring plants growing in separate containers. Typically, one container will contain a growth medium (preferably soil) and, growing thereon, one or more plants. Automated greenhouses often use sensors, timers, and computerized control systems to monitor and adjust the conditions based on specific plant requirements. The automated greenhouse of the present invention comprises a container comprising a solid material - and preferably one or more plants growing thereon - and a sensor device (300) according to the present invention. Furthermore, the automated greenhouse comprises a recorder (700). As described above, in the operation of the automated greenhouse an emitter (500) emits electromagnetic radiation to a container comprising a sensor device (300) of the present invention located in a power transmittable range. A measurement of the moisture content of the solid material is performed by the sensor device (300) of the present invention as described above and a corresponding digital data signal is emitted (710) to the recorder (700). Preferably, a reader (800) is used to both perform wireless power transfer (520) to charge the sensor device (300) and receive the emitted digital data signal (710) of the sensor device (300). An example of a reader is an RFID reader or an NFC reader.

Preferably, the automated greenhouse of the present invention comprises a watering station and a computing node wherein a container is irrigated using the watering station when the computing node has decided, based on sensor data received from the recorder (700), that the solid material needs watering. Thus, the present invention facilitates watering plants in an automated greenhouse in a reliable manner, with minimal obstruction to visual inspection of the plants growing in containers therein, and with minimal energy consumption. Preferably, a reader (800) is used to both perform wireless power transfer (520) to charge the sensor device (300) and receive the emitted digital data signal (710) of the sensor device (300). An example of a reader is an RFID reader or an NFC reader.

## Claims

1. A sensor device (100), the sensor device comprising:
an antenna (110) configured to receive electromagnetic radiation,
a capacitor (120) configured to store power received from the antenna,
a threshold activation element (130) electrically connected to the capacitor and configured to compare the stored power to a predefined threshold, wherein the predefined threshold is sufficient to enable the generation of time domain reflectometry (TDR) sensor data;
a detection element (140) configured to detect the interruption of the power supply, and
a TDR sensor (150) configured to generate TDR sensor data when both the threshold activation element signals that the stored power in the capacitor (120) exceeds the predefined threshold, and the detection element (140) signals that the antenna (110) has ceased receiving electromagnetic radiation.

2. The sensor device according to claim 1, further comprising an integrated circuit (210) that includes both the threshold activation element and the detection element (140).

3. The sensor device according to any of claims 1-2, further comprising a temperature sensor electrically connected to the threshold activation element.

4. The sensor device according to any of claims 1-3, wherein the TDR sensor further comprises a time-to-digital converter (TDC).

5. The sensor device according to any of claims 1-4, further comprising a transmitter (310) configured to generate a digital data signal and wherein the antenna is electrically connected to the transmitter to emit the digital data signal.

6. The sensor device according to claim 5, further comprising an integrated RFID chip or NFC chip, wherein the digital data signal transmitted by the device includes an ID signal based on the unique identifier of the RFID chip or NFC chip.

7. A sensor device operation method, comprising the steps of
i) providing a sensor device as described in any of claims 1-6,
ii) powering the sensor device by electromagnetic radiation from an emitter,
iii) determining, in the sensor device, if the sensor device is sufficiently powered to conduct a time domain reflectometry (TDR) measurement and if transmission of electromagnetic radiation to the device has been interrupted,
iv) optionally, when it is determined in step iii) that the sensor device is sufficiently powered to conduct the TDR measurement, preventing further powering of the sensor device by electromagnetic radiation from the emitter, and
v) performing a TDR measurement generating TDR sensor data if both conditions of step iii) are fulfilled or alternatively if the condition of step (iv) is fulfilled.

8. The method according to claim 7, further comprising the steps of:
the transmitter emitting a digital data signal based on the measurement data,
a recorder recording the digital data signal emitted by the transmitter,
comparing the measurement data obtained by the digital data signal with measurement data obtained with a previously recorded digital data signal of the same sensor device.

9. The method according to any of claims 7-8, wherein the sensor of the sensor device is inserted in a soil sample of a container, preferably a container for containing one or more plants.

10. An automated greenhouse, comprising
- a container, preferably a container for containing one or more plants, wherein said container comprises solid material, and a sensor device (100) of any of claims 5-6 for determining the moisture of the solid material,
- an emitter (500) and
- a recorder (700), further comprising conveyor means to bring the antenna (110) of the sensor device in the container within a power transmission distance to the emitter and signal exchange distance to the recorder.

11. The automated greenhouse of claim 10, comprising a watering station and a computing node wherein the container is irrigated using the watering station when the computing node has decided, based on the sensor data received from the recorder, that the solid material needs watering.

## Patentansprüche

1. Sensoreinrichtung (100), wobei die Sensoreinrichtung umfasst:
eine Antenne (110), die zum Empfangen elektromagnetischer Strahlung eingerichtet ist;
einen Kondensator (120), der zum Speichern von von der Antenne empfangener Energie eingerichtet ist;
ein Schwellwert-Aktivierungselement (130), das elektrisch mit dem Kondensator verbunden ist und dazu eingerichtet ist, die gespeicherte Energie mit einem vordefinierten Schwellwert zu vergleichen, wobei der vordefinierte Schwellwert ausreichend ist, um die Erzeugung von Zeitbereichsreflektometrie-(TDR)-Sensordaten zu ermöglichen;
ein Detektionselement (140), das dazu eingerichtet ist, eine Unterbrechung der Stromversorgung zu erkennen; und
einen TDR-Sensor (150), der dazu eingerichtet ist, TDR-Sensordaten zu erzeugen, wenn sowohl das Schwellwert-Aktivierungselement signalisiert, dass die im Kondensator (120) gespeicherte Energie den vordefinierten Schwellwert überschreitet, als auch das Detektionselement (140) signalisiert, dass die Antenne (110) keinen elektromagnetischen Strahlungsempfang mehr aufweist.

2. Sensoreinrichtung nach Anspruch 1, ferner umfassend einen integrierten Schaltkreis (210), der sowohl das Schwellwert-Aktivierungselement als auch das Detektionselement (140) beinhaltet.

3. Sensoreinrichtung nach einem der Ansprüche 1-2, ferner umfassend einen Temperatursensor, der elektrisch mit dem Schwellwert-Aktivierungselement verbunden ist.

4. Sensoreinrichtung nach einem der Ansprüche 1-3, wobei der TDR-Sensor ferner einen Zeit-Digital-Wandler (Time-to-Digital Converter, TDC) umfasst.

5. Sensoreinrichtung nach einem der Ansprüche 1-4, ferner umfassend einen Sender (310), der dazu eingerichtet ist, ein digitales Datensignal zu erzeugen, wobei die Antenne elektrisch mit dem Sender verbunden ist, um das digitale Datensignal auszusenden.

6. Sensoreinrichtung nach Anspruch 5, ferner umfassend einen integrierten RFID-Chip oder NFC-Chip, wobei das von der Einrichtung übertragene digitale Datensignal ein Identifikationssignal umfasst, das auf der eindeutigen Kennung des RFID- oder NFC-Chips basiert.

7. Verfahren zum Betrieb einer Sensoreinrichtung, umfassend die Schritte:
i) Bereitstellen einer Sensoreinrichtung gemäß einem der Ansprüche 1-6;
ii) Versorgen der Sensoreinrichtung mit Energie durch elektromagnetische Strahlung eines Senders;
iii) Bestimmen in der Sensoreinrichtung, ob die Sensoreinrichtung ausreichend mit Energie versorgt ist, um eine Zeitbereichsreflektometrie-(TDR)-Messung durchzuführen, und ob die Übertragung elektromagnetischer Strahlung zur Sensoreinrichtung unterbrochen wurde;
iv) optional, wenn in Schritt iii) bestimmt wird, dass die Sensoreinrichtung ausreichend mit Energie versorgt ist, um die TDR-Messung durchzuführen, Verhindern einer weiteren Energieversorgung der Sensoreinrichtung durch elektromagnetische Strahlung des Senders; und
v) Durchführen einer TDR-Messung zur Erzeugung von TDR-Sensordaten, wenn beide Bedingungen von Schritt iii) erfüllt sind oder alternativ die Bedingung von Schritt iv) erfüllt ist.

8. Verfahren nach Anspruch 7, ferner umfassend die Schritte:
Aussenden eines digitalen Datensignals durch den Sender auf Grundlage der Messdaten;
Aufzeichnen des vom Sender ausgesendeten digitalen Datensignals durch eine Aufzeichnungseinrichtung;
Vergleichen der anhand des digitalen Datensignals erhaltenen Messdaten mit Messdaten, die anhand eines zuvor aufgezeichneten digitalen Datensignals derselben Sensoreinrichtung erhalten wurden.

9. Verfahren nach einem der Ansprüche 7-8, wobei der Sensor der Sensoreinrichtung in eine Bodenprobe eines Behälters eingebracht ist, vorzugsweise eines Behälters zur Aufnahme einer oder mehrerer Pflanzen.

10. Automatisiertes Gewächshaus, umfassend:
- einen Behälter, vorzugsweise einen Behälter zur Aufnahme einer oder mehrerer Pflanzen, wobei der Behälter Festmaterial umfasst, und eine Sensoreinrichtung (100) nach einem der Ansprüche 5-6 zur Bestimmung der Feuchtigkeit des Festmaterials;
- einen Sender (500); und
- eine Aufzeichnungseinrichtung (700),
wobei das automatisierte Gewächshaus ferner Fördereinrichtungen umfasst, um die Antenne (110) der Sensoreinrichtung im Behälter in einen Energieübertragungsbereich des Senders sowie in einen Signalübertragungsbereich der Aufzeichnungseinrichtung zu bringen.

11. Automatisiertes Gewächshaus nach Anspruch 10, umfassend eine Bewässerungsstation und einen Rechenknoten, wobei der Behälter mittels der Bewässerungsstation bewässert wird, wenn der Rechenknoten auf Grundlage der von der Aufzeichnungseinrichtung empfangenen Sensordaten entschieden hat, dass das Festmaterial bewässert werden muss.

## Revendications

1. Un dispositif capteur (100), le dispositif capteur comprenant:
une antenne (110) configurée pour recevoir le rayonnement électromagnétique,
un condensateur (120) configuré pour stocker l'énergie reçue de l'antenne,
un élément d'activation de seuil (130) connecté électriquement au condensateur et configuré pour comparer la puissance stockée à un seuil prédéfini, où le seuil prédéfini suffit à permettre la génération de données de capteurs de réflectométrie dans le domaine temporel (TDR) ;
un élément de détection (140) configuré pour détecter l'interruption de l'alimentation électrique, et
un capteur TDR (150) configuré pour générer des données de capteur TDR lorsque l'élément d'activation seuil signale à la fois que la puissance stockée dans le condensateur (120) dépasse le seuil prédéfini, et que l'élément de détection (140) signale que l'antenne (110) a cessé de recevoir des rayonnements électromagnétiques.

2. Le dispositif capteur selon la revendication 1, comprenant en outre un circuit intégré (210) qui inclut à la fois l'élément d'activation de seuil et l'élément de détection (140).

3. Le dispositif capteur selon l'une des revendications 1 à 2, comprenant en outre un capteur de température connecté électriquement à l'élément d'activation seuil.

4. Le dispositif capteur selon l'une des revendications 1 à 3, où le capteur TDR comprend en outre un convertisseur temporel-numérique (TDC).

5. Le dispositif capteur selon l'une des revendications 1 à 4, comprenant en outre un émetteur (310) configuré pour générer un signal de données numérique, dans lequel l'antenne est connectée électriquement à l'émetteur pour émettre le signal de données numérique.

6. Le dispositif capteur selon la revendication 5, comprenant en outre une puce RFID intégrée ou une puce NFC, dans laquelle le signal de données numérique transmis par l'appareil inclut un signal d'identification basé sur l'identifiant unique de la puce RFID ou NFC.

7. Une méthode de fonctionnement de dispositif capteur, comprenant les étapes de
i) fournir un dispositif capteur tel que décrit dans l'une des revendications 1 à 6,
ii) alimenter le dispositif capteur par rayonnement électromagnétique provenant d'un émetteur,
iii) déterminer, dans le dispositif capteur, si celui-ci est suffisamment alimenté pour effectuer une mesure de réflectométrie (TDR) dans le domaine temporel et si la transmission du rayonnement électromagnétique vers l'appareil a été interrompue,
iv) éventuellement, lorsqu'il est déterminé à l'étape iii) que le dispositif capteur est suffisamment puissant pour effectuer la mesure TDR, empêchant ainsi une alimentation supplémentaire du dispositif capteur par rayonnement électromagnétique provenant de l'émetteur, et
v) effectuer une mesure TDR générant des données de capteur TDR si les deux conditions de l'étape iii) sont remplies ou alternativement si la condition de l'étape iv) est remplie.

8. La méthode selon la revendication 7, comprenant en outre les étapes suivantes:
l'émetteur émettant un signal de données numérique basé sur les données de mesure,
un enregistreur enregistrant le signal de données numérique émis par l'émetteur,
comparaison des données de mesure obtenues par le signal de données numériques avec les données de mesure obtenues avec un signal de données numérique précédemment enregistré du même dispositif capteur.

9. La méthode selon l'une des revendications 7 à 8, dans laquelle le capteur du dispositif capteur est inséré dans un échantillon de sol d'un contenant, de préférence un récipient destiné à contenir une ou plusieurs plantes.

10. Une serre automatisée, comprenant
- un contenant, de préférence un récipient destiné à contenir une ou plusieurs plantes, dans lequel ce récipient est constitué de matière solide, et un dispositif capteur (100) de l'une des revendications 5 à 6 pour déterminer l'humidité du matériau solide
- un émetteur (500) et
- une flûte à bec (700),
comprenant en outre le convoyeur permet de ramener l'antenne (110) du dispositif capteur dans le conteneur à une distance de transmission d'énergie jusqu'à l'émetteur et une distance d'échange de signal jusqu'à l'enregistreur.

11. La serre automatisée de la revendication 10, comprenant une station d'arrosage et un nœud de calcul où le récipient est irrigué à l'aide de la station d'arrosage lorsque le nœud de calcul a décidé, sur la base des données capteurs reçues de l'enregistreur, que le matériau solide a besoin d'être arrosé.
